(19) Europäisches Patentamt
European Patent Office
Office européen des brevets

(11) EP 1 364 029 B1

# (12) EUROPEAN PATENT SPECIFICATION

(45) Date of publication and mention of the grant of the patent:
**28.12.2005 Bulletin 2005/52**

(21) Application number: **02711847.0**

(22) Date of filing: **08.02.2002**

(51) Int Cl.[7]: **C12N 15/62**, C12N 15/70, C07K 14/62, C07K 14/815, C12N 1/21, C12P 21/02, C12P 21/06

(86) International application number:
**PCT/EP2002/001307**

(87) International publication number:
**WO 2002/068660 (06.09.2002 Gazette 2002/36)**

## (54) FUSION PROTEIN comprising hirudin and proinsulin or insulin

FUSIONSPROTEINE aus hirudin und proinsulin oder insulin

PROTEINE DE FUSION comprenante hirudin et proinsuline or insuline

(84) Designated Contracting States:
**AT BE CH CY DE DK ES FI FR GB GR IE IT LI LU MC NL PT SE TR**

(30) Priority: **20.02.2001 DE 10108212**

(43) Date of publication of application:
**26.11.2003 Bulletin 2003/48**

(73) Proprietor: **Sanofi-Aventis Deutschland GmbH 65929 Frankfurt am Main (DE)**

(72) Inventors:
- **HABERMANN, Paul 65817 Eppstein (DE)**
- **ERTL, Johann 65520 Bad Camberg (DE)**

(56) References cited:
**EP-A- 0 158 564**
**EP-A- 0 511 393**
**WO-A-02/04486**
**WO-A-91/09125**

- **WINTER J ET AL: "Increased production of human proinsulin in the periplasmic space of Escherichia coli by fusion to DsbA" JOURNAL OF BIOTECHNOLOGY, ELSEVIER SCIENCE PUBLISHERS, AMSTERDAM, NL, vol. 84, no. 2, 30 November 2000 (2000-11-30), pages 175-185, XP004220615 ISSN: 0168-1656**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 1 364 029 B1

SEQUENCE LISTING

<110> Aventis Pharma Deutschland GmbH

<120> Fusion protein for the secretion of a protein of interest into bacterial supernatants

<130> DEAV2001/0009

<140> 10108212.6
<141> 2001-02-20

<160> 13

<170> PatentIn Ver. 2.1

<210> 1
<211> 5
<212> PRT
<213> Artificial Sequence

<220>
<223> Description of Artificial Sequence:pBpfu_hir

<400> 1

Gly Asn Ser Ala Arg
1 5

<210> 2
<211> 58
<212> DNA
<213> Artificial Sequence

<220>
<223> Description of Artificial Sequence:pfuf1

<400> 2

ggttctctta ttgccgctac ttctttcggc gttctggcac ttacgtatac tgactgca 58

<210> 3
<211> 31
<212> DNA
<213> Artificial Sequence

<220>

<223> Description of Artificial Sequence:insullhindlll

<400> 3

tttttaagct tcatgtttga cagcttatca t 31

<210> 4
<211> 42
<212> DNA
<213> Artificial Sequence

<220>
<223> Description of Artificial Sequence:Hir_insfl

<400> 4

atccctgagg aataccttca gggaaattcg gcacgatttg tg 42

<210> 5
<211> 42
<212> DNA
<213> Artificial Sequence

<220>
<223> Description of Artificial Sequence:Hir_insrevl

<400> 5

cacaaatcgt gccgaatttc cctgaaggta ttcctcaggg at 42

<210> 6
<211> 63
<212> DNA
<213> Artificial Sequence

<220>
<223> Description of Artificial Sequence:styfimf1

<400> 6

cggcgctgag tctcgcctta ttttctcacc tatcttttgc ctctacgtat actgactgca 60
ctg 63

<210> 7
<211> 21
<212> PRT
<213> Artificial Sequence

<220>
<223> Description of Artificial Sequence: alkaline phosphatase (signal sequence)

<400> 7

Met Lys Gln Ser Thr Ile Ala Leu Ala Leu Leu Pro Leu Leu Phe Thr
1 5 10 15

Pro Val Thr Lys Ala
20

<210> 8
<211> 63
<212> DNA
<213> Artificial Sequence

<220>
<223> Description of Artificial Sequence:alkaline phosphatase (signal sequence)

<400> 8

atgaaacagt cgaccatcgc gctggcgctg ctgccgctgc tgttcacccc ggttaccaaa 60
gcg 63

<210> 9
<211> 97
<212> DNA
<213> Artificial Sequence

<220>
<223> Description of Artificial Sequence:cloning fragment

<400> 9

tttttgaat tcatgaaaca gtcgaccatc gcgctggcgc tgctgccgct gctgttcacc 60
ccggttacca aagcggctac gtatactgac tgcactg 97

<210> 10
<211> 58
<212> DNA
<213> Artificial Sequence

<220>
<223> Description of Artificial Sequence:phoaf1

<400> 10

ctgctgccgc tgctgttcac cccggttacc aaagcggcta cgtatactga ctgcactg 58

<210> 11
<211> 54
<212> DNA
<213> Artificial Sequence

<220>
<223> Description of Artificial Sequence:phoaf2

<400> 11

tttttgaat tcatgaaaca gtcgaccatc gcgctggcgc tgctgccgct gctg 54

<210> 12
<211> 40
<212> DNA
<213> Artificial Sequence

<220>
<223> Description of Artificial Sequence:Hir_insf2

<400> 12

atccctgagg aataccttca gcgatttgtg aaccagcacc 40

<210> 13
<211> 40
<212> DNA
<213> Artificial Sequence

<220>
<223> Description of Artificial Sequence:Hir_insrev2

<400> 13

ggtgctggtt cacaaatcgc tgaaggtatt cctcagggat 40

## Description

The invention relates to fusion proteins comprising a fusion part and a protein of interest, the combination of the two proteins leading to the fusion protein being secreted into the supernatant of a bacterial host and the protein of interest being present in its correct three-dimensional structure. The gene sequence for the fusion protein is part of an expression cassette which allows expression in a bacterial host. The invention relates to a process for the fermentation, expression and work-up of such a fusion protein using the expression cassette, to a plasmid containing the expression cassette, to a bacterial host cell containing the expression cassette integrated into the chromosome and/or as a replicon, for example as a plasmid, to said fusion protein with hirudin or a derivative thereof as the fusion part, to a process for producing insulin or an insulin derivative and to the use of the expression cassette in the processes for preparing a fusion protein from hirudin or derivatives thereof and for producing insulin or an insulin derivative.

The development of optimized processes for producing pharmaceuticals on the basis of recombinant proteins represents a task which has to do justice to two points of view, if possible. First, a process ought to be as cost-effective as possible and secondly, the product ought to be of the highest purity.

In this connection, the choice of expression system determines the course of the particular production process, and it is obvious to the skilled worker that the development of novel protein-chemical techniques and the wide variety of biochemical possibilities and new combinations of known techniques always make improvements of existing processes possible.

The properties of a desired protein determine in a decisive way the choice of the host cell system used for the synthesis. Bacteria such as E. coli represent the system with the aid of which it is possible to rapidly produce proteins with crude yields of several grams in inexpensive media. The system comes in useful especially for proteins which need not be modified and which can be renatured in vitro to their biologically active form. For proteins which are needed in high quantities, such as insulin for example, expression rates leading to intracellular accumulation of the protein in the form of inclusion bodies are aimed at. After cell lysis, the protein is dissolved and then, in further process steps, folded. However, the process of folding is not quantitative. Reasons for this may be irreversible damage during inclusion body formation, corresponding damage during cell lysis and errors during folding. "Wrongly" folded or modified molecules then have to be removed in further separation steps. This has an adverse effect on production costs. In addition, traces of said molecules reappear also in the final product. Since pharmaceuticals are subject to high criteria of purity, an appropriately careful and cost-intensive purification is necessary. Owing to the favorable cost / crude yield ratio, processes allowing export by E. coli of the protein of interest in correctly folded form into the culture medium would be of desirable. However, this has been successful only in exceptional cases up until now.

The international patent application PCT/EP00/08537 describes such an exception. Synthesis and export of lepirudin, the active ingredient of the pharmaceutical Refludan®, by E. coli in gram quantities was successful when using specific signal sequences for exporting. The international patent application PCT/EP01/07333 describes a bifunctional protein composed of hirudin and hirudin derivatives and of factor Xa inhibitor from ticks and derivatives thereof. Said protein can likewise be synthesized and exported by E. coli with high yields. As an addition to this finding, it was then surprisingly found that hirudin is exported with high yields not only as a fusion protein with TAP but also as part of a fusion protein with polypeptides such as proinsulin derivatives, that it is biologically active and that surprisingly a fusion partner such as proinsulin is present in the correct three-dimensional structure. This unexpected result leads to the possibility of more cost-effective production of, for example, insulin by bacterial host/vector systems, since the step of in vitro refolding after intracellular expression, which is associated with losses in yield which are not negligible, can be dispensed with and in this way a simpler protein purification process results. Another advantage is that chaotropic aids which are added to dissolve the fusion protein in traditional processes for the production of insulin in E. coli are not required. Ecologically, this leads to less environmental pollution by avoiding the corresponding waste.

Leeches of the *Hirudo* type have developed, for example, various isoforms of the thrombin inhibitor hirudin. Hirudin has been optimized for pharmaceutical requirements by artificial variation of the molecule, for example exchange of the N-terminal amino acid (e.g. EP-A 0 324 712).

The invention includes the use of hirudin and hirudin variants for the formation of fusion proteins, for example with simian proinsulin or derivatives thereof. Particular embodiments of the invention use one of the natural hirudin isoforms (the natural isoforms together are denoted "hirudin"). Natural isoforms are, for example, Val-Val-hirudin or Ile-Thr-hirudin. Other embodiments of the invention use a variant of a natural hirudin isoform. A variant is derived from a natural hirudin isoform but contains, for example, additional amino acids and/or amino acid deletions and/or amino acid exchanges compared with the natural isoform. A hirudin variant may contain alternating peptide segments of natural hirudin isoforms and new amino acids. Hirudin variants are known and are described, for example, in DE 3 430 556. Hirudin variants are commercially available in the form of proteins (Calbiochem® Biochemicals, Cat.no.377-853, -950-960).

Insulin is a polypeptide of 51 amino acids which are distributed between two amino acid chains: the A chain with 21 amino acids and the B chain with 30 amino acids. The chains are connected to one another by 2 disulfide bridges. Insulin compositions have been used for many years for the therapy of diabetes. This includes the use not only of naturally occurring insulins but also of insulin derivatives and analogs.

Insulin derivatives are derivatives of naturally occurring insulins, namely human insulin or animal insulins, which differ from the corresponding, otherwise identical naturally occurring insulin by substitution of at least one naturally occurring amino acid residue and/or addition of at least one amino acid residue and/or organic residue.

In general, insulin derivatives have a slightly modified action compared with human insulin.

Insulin derivatives having an accelerated onset of action are described in EP 0 214 826, EP 0 375 437 and EP 0 678 522. EP 0 124 826 inter alia relates to substitutions of B27 and B28. EP 0 678 522 describes insulin derivatives which have at position B29 various amino acids, preferably proline, but not glutamic acid. EP 0 375 437 includes insulin derivatives with lysine or arginine at B28, which may additionally be modified at B3 and/or A21, where appropriate.

EP 0 419 504 discloses insulin derivatives which are protected against chemical modification by modification of asparagine at B3 and of at least one other amino acid at positions A5, A15, A18 or A21.

WO 92/00321 describes insulin derivatives in which at least one amino acid at positions B1-B6 has been replaced by lysine or arginine. According to WO 92/00321, insulins of this kind exhibit a prolonged action.

When producing insulin and insulin derivatives by genetic engineering, an insulin precursor, "proinsulin", comprising B, C and A chains is frequently expressed. Said proinsulin can be converted into insulin or an insulin derivative by enzymatic or chemical removal of the C chain after appropriate and correct folding and formation of the disulfides bridges. Proinsulin is frequently expressed in the form of a fusion protein. The "unwanted" fusion partner likewise needs be removed chemically or enzymatically.

It is obvious to the skilled worker that the choice of recombinant host/vector systems determines the methods for cultivation, propagation and fermentation of the recombinant cells. This is likewise a subject of the invention.

The fusion protein shows surprisingly good solubility in acidic medium, and this leads to distinct advantages regarding the chemical workup of the protein. Firstly, many unwanted components of the supernatant are precipitated under said conditions and, secondly, peptidases or proteases are inactive. Thus, acidifying the fermentation broth at the end of the operation makes it possible to directly separate unwanted supernatant proteins together with the host cells from the fusion protein and, in a further step, to concentrate said fusion protein. This is likewise a subject of the invention.

At the end of the fermentation, the folding process may not yet be 100% complete. The addition of mercaptan or, for example, cysteine hydrochloride can complete the process. This is likewise a subject of the invention.

If the two proteins are fused via a linker of amino acids which are specifically recognized by endoproteases which efficiently cleave the fusion protein at no other position, then the protein of interest can be cleaved off directly in active form. In the case of insulin production, the linker between hirudin and proinsulin preferably contains arginine at the carboxy-terminal end. In simultaneous processing it is then possible by conversion with trypsin to cleave off the fusion part and convert proinsulin to mono- or di-Arg-insulin. Said linker must be optimized in relation to insulin processing such that cleaving off the hirudin part is not slower than cleavages in the C peptide sequence or a derivative thereof which links the B and A chains of insulin. This is likewise a subject of the invention. An example of an expression system which can be used is the vector pJF118, described in figure 1 of European patent 0 468 539.

Plasmids which contain DNA sequences encoding proinsulin or proinsulin derivatives are described, for example, in the patents EP-A 0 489 780 and PCT/EP00/08537.

The plasmid pK152 which contains the sequence for hirudin according to EP-A 0 324 712 is used as source of the DNA sequence for hirudin.

The export compatibility of the protein of interest for passing through the inner bacterial membrane is important for secretion. In this context, the choice of signal sequence which can be more or less optimal for different proteins is important. The patent application PCT/EP00/08537 describes a system of PCR-based signal sequence screening. This system can also be applied to fusion proteins having hirudin as the N-terminal fusion part, since hirudin activity surprisingly remains intact and thus becomes readily detectable in the supernatant by means of the thrombin inhibition assay.

Winter et al. (Journal of Biotechnology 84 (2000) 175-185) describe an increased production of human proinsulin in the periplasmic space of *Escherichia coli* by fusion to DsbA.

In international patent application PCT/GB90/01911 it is disclosed that relatively inactive fusion proteins are activatable by enzymes of the clotting cascade to have fibrinolytic and/or clot formation inhibition activity. For example, a fusion protein comprising two hirudin or streptokinase molecules, linked by a cleavable linkage sequence, may be cleaved to yield anti-thrombotic hirudin or fibrinolytic streptokinase by thrombin or Factor Xa.

In EP 0 158 564 A1 vectors for the expression of hirudin are disclosed.

The invention therefore relates to a DNA (alternative term: expression cassette) encoding a fusion protein of the form

$$- F - As_m - R_n - Y -,$$

where

- F is a DNA sequence coding for an amino acid sequence which allows secretion of a protein Y into a fermentation medium, wherein F encodes a hirudin,
- As is a chemical bond or a DNA sequence coding for an amino acid encodable by the genetic code,
- m is an integer from 0 - 10,
- R is a chemical bond or an arginine codon,
- n is 0 or 1, and
- Y is a DNA sequence coding for a protein of interest which, correctly folded, is part of the fusion protein in the fermentation medium and which is a proinsulin.

The invention further relates to an expression cassette (alternative term: DNA-molecule) of the form

$$P - S - F - As_m - R_n - Y - T,$$

where

- P is a promoter,
- S is a DNA sequence coding for a signal sequence allowing optimal yields,
- T is an untranslated expression-enhancing DNA sequence.

The invention further relates to a plasmid containing an above-described expression cassette and to a host cell containing said plasmid or to a host cell which preferably contains the expression cassette integrated into the host genome, the host cell being selected from a group comprising E.coli, B. subtilis and Streptomyces.

The invention also relates to a process for the fermentative production of a fusion protein as described above, in which process

(a) an DNA molecule as described above is expressed in a host cell as described above and
(b) the expressed fusion protein is isolated;

in which, in particular, the supernatant is separated from the host cells to isolate the expressed protein, and the expressed protein is isolated from the supernatant; and in which a process step for concentrating the expressed protein in the supernatant after precipitation is selected from a group comprising microfiltration, hydrophobic interaction chromatography and ion exchange chromatography, and in which a particular embodiment is that isolation of the expressed protein includes a step in which components of the culture medium or the supernatant are precipitated, while the expressed protein remains in solution; and in which in a further preferred embodiment of the invention, after the fermentation, mercaptan or cysteine hydrochloride are added to the fermentation supernatant at pH 6 - 9, resulting in a free SH group concentration of from 0.05 to 2.5 mM.

A particular embodiment of the invention comprises separating the fermentation supernatant from the host cells, further culturing the host cells in fresh medium and isolating the released fusion protein from the supernatant. In other words, a further embodiment of the invention is a process as described above, in which process after separating the fermentation supernatant from the host cells, the host cells are repeatedly cultured in fresh medium, and the released fusion protein is isolated from each supernatant obtained during cultivation.

The invention further relates to a process for the production of insulin, in which process

(a) from the expressed fusion protein which is obtained in a process as described above
(b) insulin, is released by enzymatic or chemical cleavage and
(c) is isolated.

The following examples which are not intended to be restrictive describe the invention in more detail.

Example 1: Construction of a lepirudin-GNSAR-simian proinsulin fusion protein, appended to the signal sequence of the oprF gene product from Pseudomonas fluorescens.

Example 2 of the patent application PCT/EP00/08537 described an expression vector which allows expression and secretion of Refludan into the medium used for E. coli via the signal sequence of the Pseudomonas fluorescens oprF gene product (De, E. et al. FEMS Microbiol Lett. 127,263 -272, 1995). This vector serves to construct a Refludan-GNSAR-simian proinsulin fusion protein (GNSAR=SEQ ID NO.: 1) and is denoted pBpfu_hir.
Further starting materials are pJF118 (EP 0 468 539) and pK152 (PCT/EP00/08537) plasmid DNAs. The following oligonucleotides are required:

Primer pfuf1

**5´GGTTCTCTTA TTGCCGCTAC TTCTTTCGGC GTTCTGGCAc ttacgtatac tgactgca 3´ (SEQ ID NO.: 2)**

Primer insu11hindIII

**5´ - TTTTTAAGCT TCATGTTTGA CAGCTTATCA T –3´ (SEQ ID NO.: 3)**

Primer Hir_insf1

5´ ATCCCTGAGG AATACCTTCA G**GGAAATTCG GCACGATTTG TG** – 3´(SEQ ID NO.: 4)

Primer Hir_insrev1

5´ - **CACAAATCGT GCCGAATTTC C**CTGAAGGTA TTCCTCAGGG AT –3´(SEQ ID NO.: 5)

Primer pfuf1 hybridizes with the DNA region encoding the junction of signal sequence and lepirudin in the expression vector.
The part of primer Hir_insrev1 shown in bold type hybridizes with the DNA region encoding the junction of preproinsulin and simian proinsulin sequences in plasmid pINT90d and with sequences of the 3' end of the hirudin sequence in plasmid pK152. Primer Hir_insrev1 is 100% complementary to primer Hir_insf1. Primer Insu11HindIII marks the 3' end of the DNA region cloned in pINT90d and encoding the simian proinsulin sequence and additionally carries the hexanucleotide sequence for recognition by the restriction enzyme HindIII.
Two standard polymerase chain reactions are carried out using the Hir_insf1 / Insu11HindIII primer pair with plasmid pINT90d as template and the pfuf1/ Hir_insrev primer pair with plasmid pBpfu_hir as template. The products of both reactions are combined and an aliquot is converted in a third polymerase chain reaction with primers pfuf1/Insu11HindIII. The result is a DNA product which contains the sequence signal (partially)-lepirudin-GNSAR- simian proinsulin. The DNA fragment is converted using restriction enzymes BamHI and HindIII, with BamHI cleaving in the lepirudin sequence and HindIII at the 3' end of the proinsulin-encoding sequence.
In a parallel reaction, vector pBpfu is converted using the two enzymes and the large vector fragment is isolated. The isolated products of both reactions are converted in a T4 ligase reaction. Competent cells of the E. coli strain K12 Mc1061 (Sambrook et al. "Molecular Cloning" (Cold Spring Habor Laboratory Press 1989) are transformed with the ligation mixture and plated on NA plates containing 25µg /ml ampicillin. Plasmid DNA is isolated from transformants for characterization. At the same time, a plate with the transformants characterized in the plasmid analysis is produced for maintenance purposes. The DNA is characterized by means of restriction analysis and DNA sequence analysis. A plasmid identified as correct was denoted pBpfuHir_Ins.

Example 2: Construction of a Ser-hirudin-GNSAR-simian proinsulin fusion protein appended to the signal sequence of S. typhimurium outer membrane protein (fimD)

The construction is carried out according to the plan described in example 1.
Example 10 of patent application PCT/EP 00/08537 describes the construction of a vector for exporting lepirudin via the signal sequence of S. typhimurium outer membrane protein (Rioux,C.R., Friedrich,M.J. and Kadner,R. J.;J. Bacteriol. 172 (11), 6217-6222 (1990)) . The resulting plasmid is denoted pBstyfim_hir for laboratory purposes. DNAs of plasmids pK152 and pINT90d serve in each case as templates.
The construction requires 4 primers.
The primers insu11HindIII, Hir_insf1 and Hir_insrev1 are described in example 1.
The primer styfimf1ser is newly synthesized and has the following sequence:

5´ CGGCGCTGAG TCTCGCCTTA TTTTCTCACC TATCTTTTGC C**TCT**acgtat actgactgcaCTG 3´ (SEQ ID NO.: 6)

The DNA triplet shown in bold type indicates a serine codon. As a result, a hirudin is produced which carries serine instead of leucine at position 1 of the amino acid sequence.
Corresponding to example 1, two standard polymerase chain reactions are carried out using the Hir_insf1 / Insu11HindIII primer pair with pINT90d DNA as template and the styfimf1ser / Hir_insrev primer pair with pK152 DNA as template. The products of both reactions are combined and an aliquot is converted in a third polymerase chain reaction with primers styfimf1 ser /Insu11HindIII. The result is a DNA product which contains the sequence signal (partially)-Ser-hirudin-GNSAR- simian proinsulin. The DNA fragment is converted using the restriction enzymes BamHI and HindIII.
In a parallel reaction, vector pBstyfim_Hir is converted using the two enzymes and the large vector fragment is isolated. The isolated products of both reactions are converted in a T4 -ligase reaction. Competent cells of E. coli strain K12 Mc1061 are transformed with the ligation mixture, and plasmid DNA is isolated from transformants for characterization. At the same time, a plate with the transformants characterized by plasmid analysis is produced for maintenance purposes. The DNA is characterized by means of restriction analysis and DNA sequence analysis. A plasmid identified as correct was denoted pBstyfim_SerHir_Ins.

Example 3: Construction of an Ala-hirudin-R-simian proinsulin fusion protein appended to the signal sequence of the E. coli alkaline phosphatase precursor protein

The E. coli alkaline phosphatase precursor has the signal sequence:

MKQSTIALAL LPLLFTPVTK A (SEQ ID NO.: 7)

(Shuttleworth H., Taylor J., Minton N.; Nucleic Acids Res. 14:8689, (1986)).
The peptide sequence is translated into DNA by the GCG program Backtranslate (Wisconsin Package Version 10.1, Genetics Computer Group (GCG), Madison, Wisc.) using the E. coli high codon usage criteria.
This results in the sequence:

5´ATGAAACAGTCGACCATCGCGCTGGCGCTGCTGCCGCTGCTGTTCACCCCGGT TACCAAAGCG 3´ (SEQ ID NO.: 8)

To clone and append this sequence to a DNA sequence coding for a hirudin which is characterized by having the amino acid alanine at position 1 (EP-A 0 448 093), said sequence is extended by the sequence shown in bold type:

5'**TTTTTTGAATTC**ATGAAACAGTCGACCATCGCGCTGGCGCTGCTGCCGCTGCTGTTCAC CCCGGTTACCAAAG -CG **GCT****acgtat actgactgcaCTG** (SEQ ID NO.: 9)

Two oligonucleotide sequences which partially overlap are derived therefrom.

Primer phoaf1 has the sequence :

**5'CTGCTGCCGCTGCTG**TTCACCCCGGTTACCAAAGCG GCTACG TATACTGACTGCACTG –3' (SEQ ID NO.: 10)

Primer phoaf2 has the sequence:

5'TTTTTTGAATTCATGAAACAGTCGACCATCGCGCTGGCG**CTGCTGCCGCTGCTG** –3' (SEQ ID NO.: 11)

The construction of the expression vector requires primers insu11HindIII , Hir_insf2 and Hir_insrev2 and DNAs of plasmids pK152, pINT90d and pJF118.

Primer Hir_insf2 has the sequence:

5' - ATCCCTGAGGAATACCTTCAG**cga****TTTGTGAACCAGCAC C** -3'(SEQ ID NO. 12)

Primer Hir_insrev2 has the sequence:

5' - **GGTGCTGGTTCACAAA****tcg**CTGAAGGTA TTCCTCAGGG AT-3'(SEQ ID NO.13)

Upper case letters in bold type indicate the sequence hybridizing with proinsulin, while upper case letters in plain type describe overlap with the 3' end of the hirudin sequence. Lower case letters underlined and in bold type represent the codon for the linker arginine.

Corresponding to example 1, two standard polymerase chain reactions are carried out using the Hir_insf1 / Insu11HindIII primer pair with pINT90d DNA as template and the phoaf1 / Hir_insrev primer pair with pK152 DNA as template. The products of both reactions are combined and an aliquot is converted in a third polymerase chain reaction with primers phoa /Insu11HindIII. The result is a DNA product which contains the sequence signal-Ala-hirudin-GNSAR-simian proinsulin. The DNA fragment is converted using restriction enzymes BamHI and HindIII. In a parallel reaction, vector pjF118 is converted using the two enzymes and the large vector fragment is isolated. The isolated products of both reactions are converted in a T4-ligase reaction. Competent cells of E. coli strain K12 Mc1061 are transformed with the ligation mixture, and plasmid DNA is isolated from transformants for characterization. At the same time, a plate with the transformants characterized by plasmid analysis is produced for maintenance purposes. The DNA is characterized by means of restriction analysis and DNA sequence analysis. A plasmid identified as correct was denoted pNS22.

Example 4: Thrombin inhibition assay

The hirudin concentration is determined according to the method of Grießbach et al. (Thrombosis Research 37, pp. 347 -350 , 1985). For this purpose, specific amounts of a Refludan standard are included in the measurements in order to establish a calibration curve from which the yield in mg/l can be determined directly. The biological activity is also a direct measure for correct folding of the proinsulin component of the fusion protein. Alternatively, it is possible to use a proteolytic S. aureus digestion and subsequent analysis in an RP-HPLC system to determine the correct S-S bridge formation.

Example 5: Expression of the fusion protein

Recombinant cells are cultivated overnight in 2YT medium (per liter: 16 g of tryptone, 10 g of yeast extract, 5 g of NaCl) containing 100 μg/ml ampicillin. The overnight culture is diluted 1:50 with fresh medium and the cells are cultivated to a density of approximately 0.8 $OD_{600}$.

Expression is then induced by adding IPTG in such a way that a concentration of 0.05-2 mM is established. The cells induced in this way are incubated for a further 3-26 h.

After three hours, an antithrombin action of hirudin is clearly measurable in the supernatant. Said action can be attributed to secretion of the desired fusion protein, since SDS PAGE analysis, after Coomassie blue staining, reveals only in induced cells a new band which reacts in Western blot analysis with polyclonal anti-insulin antibodies. In fermentation experiments, induction is commenced only after cultivation to significantly higher optical densities. Preference is given here to synthetic media based on minimal medium.

Cell productivity can be increased by using the principle of bacterial milking, i.e. by carefully removing the cells, after the optimal induction time, from the supernatant and further incubating them in fresh medium to which the inducer can again be added. Insulin is then prepared in parallel from the harvested supernatant.

Example 6: Purification of the fusion protein

After induction has finished, the cell supernatant is adjusted to pH 2.5 - 3 and cells and supernatant components are removed by centrifugation or filtration. The supernatant of the precipitation is applied to a cation exchange column (S - Hyper DF, Source 30S) and fractionated using a linear gradient from 150 to 450 mM NaCl at pH 3.5 in the presence of 30% 2-propanol. The individual fractions are analyzed by means of RP-HPLC. The proinsulin-hirudin fusion protein is eluted at an NaCl concentration of about 300 mM. Sufficiently pure fractions are combined, diluted with 0,1% TFA and applied to an RP column (PLRP -S 7.5 x 50 mm) by pumping. Elution is carried out using a gradient of 25-50% acetonitrile. Two groups of fractions are pooled. After removing the solvent, the material is freeze-dried. The purity of the material is checked by means of SDS polyacrylamide electrophoresis. The purified fusion protein is analyzed by mass spectrometry (ESI). The experimentally determined molecular weight of the fusion protein corresponds to its theoretically expected molecular weight after removal of the signal peptide.

Example 7: Determination of the disulfide bridge linkage

The fusion protein is digested with trypsin and the fragments formed are analyzed by means of RP-HPLC and subsequently by means of mass spectrometry. A fragment which is recognized as de-(B30) insulin, due to its mass of 5706 Da, is successfully identified. This product is subjected to S. aureus V8 protease digestion. RP-HPLC analysis shows the expected peptide pattern.

Trypsin cleavage is carried out as follows:

The freeze-dried fusion protein is dissolved in 50 mMTris-HCl pH 8 (1 mg/ml), and trypsin (1 μg per mg of fusion protein) is added. Trypsin is inactivated at pH 3 at the end of the reaction.

The S. aureus digestion is carried out as follows:

The isolated de-(B30) insulin is dissolved in water at pH 8, S. aureus protease (1/50 of the amount of insulin) is added, and the mixture is incubated at 37°C for 5 hours and then at room temperature overnight.

Example 8: Purification of insulin

In contrast to most other polypeptides found in the supernatant due to either spontaneous lysis of host cells or secretion, the fusion protein is surprisingly not precipitated at pH 2.5-3,5. The culture medium is therefore acidified appropriately and then, after completion of the precipitation, the precipitate and the cells are removed by centrifugation or by microfiltration and concentrated.

Subsequently, the medium is adjusted to pH 6.8 and the fusion protein content is determined in parallel by analytical HPLC measurement. The determination is followed by adding trypsin to the supernatant so that trypsin is at approx. 1 µg per 1-1.5 mg of fusion protein. After incubation at room temperature for approx. 4 hours, purification is carried out by cation exchange chromatography at pH 3.5 in the presence of 2-propanol. Elution is carried out in the buffer by applying a gradient of from 0.15 to 0.45 M.

Di-Arg-insulin is eluted at approx. 0.3 M. After 1:1 dilution, di-Arg-insulin is precipitated from the insulin-containing fractions at pH 6.8 with the addition of a 10% strength $ZnCl_2$ solution. Insulin is filtered off and then dissolved in 0.05 M Tris-HCl (pH 8.5) resulting in a 2 mg/ml solution:
Then the amount of approximately 1 unit of carboxypeptidase B per 100ml solution is added and the reaction is carried out with gentle stirring. The pH is then adjusted to pH 5.5 with citric acid, and insulin is crystallized in the presence of $ZnCl_2$. The crystals are removed, dissolved and, after purification by RP-HPLC, insulin is purified again by crystallization.

Example 9: Processing of the fusion protein directly in the culture medium

At the end of the expression period, the culture medium is adjusted to pH 6.8 and trypsin is then added with stirring so that a final concentration of 4-8 mg per liter is established. After incubation for approx. 4 hours, the fermentation broth treated in this way is adjusted to pH 2.5-3. After 1-6 hours of precipitation, the pH is raised to 3.5, and the di-Arg-insulin formed is purified via cation exchange chromatography in the presence of 30% 2-propanol. Elution is carried out by means of an NaCl gradient of 0.05-0.5 M salt. The product-containing fractions are diluted 1:1 with $H_2O$ and then $ZnCl_2$ is added, so that a 0.1 % strength $ZnCl_2$ solution is formed. Di-Arg-insulin precipitates at pH 6.8 and by way of example is converted to insulin according to example 8.

Example 10: Further signal sequences for the secretion of fusion proteins

Using the technique described by the patent application PCT/EP00/08537 further signal sequences leading to the secretion of hirudin - proinsulin fusion protein could be detected :

Signal sequence smompa derived from the ompA gene for major outer membrane protein of Serratia marcescens (GenEMBL data base locus: SMOMPA, 1364 bp DNA BCT 30-MAR-1995)

Signal sequence ecoompc derived from E.coli ompC gene coding for major outer membrane protein (GenEMBL data base locus : SMOMPA,1364bp,DNA BCT 30-MAR-1995)

Signal sequence af009352 derived from Bacillus subtilis osmoprotectant binding protein precursor (opuCC) (GenEMBL data base locus: AF009352, 4500bp, DNA BCT 23-JUL-1997)

Signal sequence aeoxyna derived from Aeromonas caviae xynA gene for xylanase I precursor (GenEMBL data base locus : AEOXYNA,1139bp, DNA BCT 07-FEB-1999)

Signal sequence stomps1 derived from S.typhi gene for outer membrane protein S1 ( GenEMBL data base locus : STOMPS1,1938 bp, DNA BCT 24-AUG-1995)

## Claims

**1.** A DNA coding for a fusion protein of the form

$$-F-AS_m-R_n-Y-$$

where

- F is a DNA sequence coding for an amino acid sequence which allows secretion of a protein Y into a fermentation medium, wherein F encodes a hirudin,
- As is a chemical bond or a DNA sequence coding for an amino acid encodable by the genetic code,
- m is an integer from 0 - 10,
- R is a chemical bond or an arginine codon,
- n is 0 or 1, and
- Y is a DNA sequence coding for a protein of interest which, correctly folded, is part of the fusion protein in the fermentation medium and which is a proinsulin.

**2.** A DNA according to claim I, wherein the expression cassette is of the form

$$P - S - F - AS_m - R_n - Y - T$$

where

- P is a promoter,
- S is a DNA sequence coding for a signal sequence allowing optimal yields,
- T is an untranslated expression-enhancing DNA sequence.

**3.** A DNA according to claim 2, wherein S is the oprF gene from Pseudomonas fluorescens, the DNA encoding the signal sequence of S. typhimurium outer membrane protein (fim D), the DNA sequence encoding the signal sequence of the E. coli alkaline phosphatase precursor protein, the DNA sequence encoding the signal sequence smompa derived from the ompA gene for major outer membrane protein of Serratia marcescens, the DNA sequence encoding the signal sequence ecoompc derived from E.coli ompC gene coding for major outer membrane protein, the DNA sequence encoding the signal sequence af009352 derived from Bacillus subtilis osmoprotectant binding protein precursor (opuCC), the DNA sequence encoding the signal sequence aeoxyna derived from Aeromonas caviae xynA gene for xylanase I precursor, or the DNA sequence encoding the signal sequence stomps1 derived from S.typhi gene for outer membrane protein S1.

**4.** A DNA according to any of claims 2 or 3, wherein the DNA sequence F encodes lepirudin or a hirudin which carries serine or alanine instead of leucine at position 1 of the amino acid sequence.

**5.** A fusion protein encoded by a DNA according to any of the claims 1 to 4.

**6.** A plasmid comprising a DNA according to any of claims 1 to 4.

**7.** A host cell comprising a plasmid according to claim 6.

**8.** A host cell comprising a DNA according to any of claims 1 to 4.

**9.** A host cell according to claims 7 or 8, wherein the cell is selected from the group comprising E. coli, B.subtilis and Streptomyces.

**10.** A host cell according to claim 9, wherein the plasmid according to claim 6 or the DNA according to any of claims 1 to 4 is integrated in the genome of the host cell.

**11.** A process for the fermentative production of a fusion protein in which process

(a) a DNA molecule according to any of claims 1 to 4 is expressed in a host cell according to any of claims 7 to 9; and
(b) the expressed fusion protein is isolated.

**12.** A process according to claim 11, wherein the supernatant is separated from the host cell to isolate the expressed protein, and the expressed protein is isolated from the supernatant.

**13.** A process according to any of the claims 11 or 12, wherein a process step for concentrating the expressed protein in the supernatant after precipitation is selected from a group comprising microfiltration, hydrophobic interaction chromatography and ion exchange chromatography.

**14.** A process according to any of claims 11 to 13, wherein isolation of the expressed protein includes a step in which components of the culture medium or the supernatant are precipitated, while the expressed protein remains in solution.

**15.** A process according to any of claims 11 to 14, wherein after the fermentation, mercaptan or cysteine hydrochloride are added to the fermentation supernatant at pH 6-9, resulting in a free SH group concentration from 0.05 to 2.5 mM.

**16.** Process according to any of claims 11 to 15, in which process after separating the fermentation supernatant from the host cells, the host cells are repeatedly cultured in fresh medium, and the released fusion protein is isolated from each supernatant obtained during cultivation.

**17.** Process according to any of claims 11 to 15 in which mercaptane or cystein hydrochloride is added to the supernatant of the cell culture at pH 6 - 9, so that a concentration of 0,05 to 2,5 mM of free SH-groups is reached.

**18.** A process for the production of insulin, in which process

(a) from the expressed fusion protein which is obtained in a process according to any of claims 11 to 17,
(b) insulin is released by enzymatic or chemical cleavage and
(c) is isolated.

**Patentansprüche**

**1.** DNA, codierend für ein Fusionsprotein der Formel

$$-F-As_m-R_n-Y-$$

worin

F eine DNA-Sequenz ist, die für eine Aminosäuresequenz codiert, welche die Sekretion eines Proteins Y in ein Fermentationsmedium erlaubt, worin F für ein Hirudin steht,
As eine chemische Bindung oder eine DNA-Sequenz ist, die für eine Aminosäure codiert, die durch den genetischen Code codierbar ist,
m eine ganze Zahl von 0 bis 10 ist,
R eine chemische Bindung oder ein Arginincodon ist,
n 0 oder 1 ist, und
Y eine DNA-Sequenz ist, die für ein Protein von Interesse codiert, welches korrekt gefaltet Teil des Fusionsproteins im Fermentationsmedium ist und welches ein Proinsulin ist.

**2.** DNA nach Anspruch 1, wobei die Expressionskassette die folgende Form hat:

$$P\text{-}S\text{-}F\text{-}AS_m\text{-}R_n\text{-}Y\text{-}T$$

worin

P ein Promotor ist,
S eine DNA-Sequenz ist, die für eine Signalsequenz codiert, welche optimale Ausbeuten ermöglicht,
T eine nicht translatierte Expressions-verstärkende DNA-Sequenz ist.

**3.** DNA nach Anspruch 2, wobei S das oprF-Gen aus Pseudomonas fluorescens, die DNA, die für die Signalsequenz von S. typhimurium-Außenmembranprotein (fim D) codiert, die DNA-Sequenz, die für die Signalsequenz des alkalische Phosphatase-Vorläuferproteins von E. coli codiert, die DNA-Sequenz, die für die Signalsequenz smompa, abgeleitet vom ompA-Gen für das Hauptaußenmembranprotein von Serratia marcescens, codiert, die DNA-Sequenz, die für die Signalsequenz ecoompc, abgeleitet vom E. coli-ompC-Gen, das für das Hauptaußenmembranprotein codiert, codiert, die DNA-Sequenz, die für die Signalsequenz af009352, abgeleitet vom Bacillus subtilis-Osmoprotectant-Bindungsproteinvorläufer (opuCC), codiert, die DNA-Sequenz, die für die Signalsequenz aeoxyna, abgeleitet vom Aeromonas caviae-xynA-Gen für Xylanase I-Vorläufer, codiert, oder die DNA-Sequenz, die für die Signalsequenz stomps1, abgeleitet vom S.typhi-Gen für das Außenmembranprotein S1, codiert.

**4.** DNA nach einem der Ansprüche 2 oder 3, wobei die DNA-Sequenz F für Lepirudin oder ein Hirudin, das Serin oder Alanin anstelle von Leucin in Position 1 der Aminosäuresequenz trägt, codiert.

**5.** Fusionsprotein, das durch eine DNA nach einem der Ansprüche 1 bis 4 codiert wird.

**6.** Plasmid, das eine DNA nach einem der Ansprüche 1 bis 4 umfasst.

**7.** Wirtszelle, die ein Plasmid nach Anspruch 6 umfasst.

**8.** Wirtszelle, die eine DNA nach einem der Ansprüche 1 bis 4 umfasst.

**9.** Wirtszelle nach Anspruch 7 oder 8, wobei die Zelle aus der Gruppe ausgewählt ist, die E. coli, B. subtilis und Streptomyces umfasst.

**10.** Wirtszelle nach Anspruch 9, wobei das Plasmid nach Anspruch 6 oder die DNA nach einem der Ansprüche 1 bis 4 in das Genom der Wirtszelle integriert ist.

**11.** Verfahren zur fermentativen Produktion eines Fusionsproteins, wobei das Verfahren umfasst:

(a) ein DNA-Molekül nach einem der Ansprüche 1 bis 4 wird in einer Wirtszelle nach einem der Ansprüche 7 bis 9 exprimiert und

(b) das exprimierte Fusionsprotein wird isoliert.

**12.** Verfahren nach Anspruch 11, wobei der Überstand von der Wirtszelle unter Isolierung des exprimierten Proteins abgetrennt wird und das exprimierte Protein aus dem Überstand isoliert wird.

**13.** Verfahren nach irgendeinem der Ansprüche 11 oder 12, wobei ein Verfahrensschritt zur Konzentrierung des exprimierten Proteins im Überstand nach Präzipitation aus einer Gruppe, umfassend Mikrofiltration, hydrophobe Wechselwirkungschromatographie und Ionenaustauschchromatographie, ausgewählt wird.

**14.** Verfahren nach irgendeinem der Ansprüche 11 bis 13, wobei eine Isolierung des exprimierten Proteins einen Schritt umfasst, in dem Komponenten des Kulturmediums oder der Überstand präzipitiert werden, während das exprimierte Protein in Lösung verbleibt.

**15.** Verfahren nach einem der Ansprüche 11 bis 14, wobei nach der Fermentation Mercaptan oder Cysteinhydrochlorid zu dem Fermentationsüberstand mit einem pH 6-9 gegeben werden, was in einer Konzentration an freier SH-Gruppe von 0,05 bis 2,5 mM resultiert.

**16.** Verfahren nach irgendeinem der Ansprüche 11 bis 15, wobei in dem Verfahren nach Abtrennung des Fermentationsüberstands von den Wirtszellen die Wirtszellen wiederholt in frischem Medium kultiviert werden und das freigesetzte Fusionsprotein aus jedem Überstand, der während der Kultivierung erhalten wird, isoliert wird.

**17.** Verfahren nach irgendeinem der Ansprüche 11 bis 15, wobei Mercaptan oder Cysteinhydrochlorid zu dem Überstand der Zellkultur mit pH 6-9 gegeben wird, so dass eine Konzentration von 0,05 bis 2,5 mM an freien SH-Gruppen erreicht wird.

**18.** Verfahren für die Herstellung von Insulin, wobei das Verfahren umfasst:

(a) aus dem exprimierten Fusionsprotein, das in einem Verfahren nach einem der Ansprüche 11 bis 17 erhalten wird,

(b) wird Insulin durch enzymatische oder chemische Spaltung freigesetzt und

(c) wird isoliert.

**Revendications**

**1.** ADN codant pour une protéine de fusion de la forme :

$$-F-As_m-R_n-Y-$$

où

- F est une séquence d'ADN codant pour une séquence d'acides aminés qui permet la sécrétion d'une protéine Y dans un milieu de fermentation, dans laquelle F code pour une hirudine,
- As est une liaison chimique ou une séquence d'ADN codant pour un acide aminé pouvant être codé par le code génétique,
- m est un entier de 0 à 10,
- R est une liaison chimique ou un codon d'arginine,
- n est 0 ou 1 et
- Y est une séquence d'ADN codant pour une protéine d'intérêt laquelle, correctement repliée, fait partie de la protéine de fusion dans le milieu de fermentation et est une proinsuline.

**2.** ADN selon la revendication 1, dans lequel la cassette d'expression est de la forme :

$$P - S - F - AS_m - R_n - Y - T$$

où

P est un promoteur,
S est une séquence d'ADN codant pour une séquence signal permettant d'obtenir des rendements optimaux,
T est une séquence d'ADN non traduite amplificatrice d'expression.

**3.** ADN selon la revendication 2, dans lequel S est : le gène oprF provenant de *Pseudomonas fluorescens ;* l'ADN codant pour la séquence signal de la protéine de la membrane plasmique de *S. typhimurium* (fim D) ; la séquence d'ADN codant pour la séquence signal de la protéine précurseur de la phosphatase alcaline d'*E. coli* ; la séquence d'ADN codant pour la séquence signal "smompa" dérivée du gène ompA pour la principale protéine de la membrane plasmique de *Serratia marcescens* ; la séquence d'ADN codant pour la séquence signal "ecoompc" dérivée du gène ompC provenant d'E. coli codant pour la principale protéine de la membrane plasmique ; la séquence d'ADN codant pour la séquence signal "af009352" dérivée du précurseur de la protéine de liaison osmo-protectrice de *Bacillus subtilis* (opuCC) ; la séquence d'ADN codant pour la séquence signal "aeoxyna" dérivée du gène xynA d'*Aeromonas caviae* pour le précurseur de la xylanase I ; ou la séquence d'ADN codant pour la séquence signal "stomps1" dérivée du gène de *S. typhi* pour la protéine S1 de la membrane plasmique.

**4.** ADN selon l'une quelconque des revendications 2 ou 3, dans lequel la séquence d'ADN F code pour une lépirudine ou une hirudine, qui porte une sérine ou une alanine au lieu d'une leucine à la position 1 de la séquence d'acides aminés.

**5.** Protéine de fusion codée par un ADN selon l'une quelconque des revendications 1 à 4.

**6.** Plasmide comprenant un ADN selon l'une quelconque des revendications 1 à 4.

**7.** Cellule hôte comprenant un plasmide selon la revendication 6.

**8.** Cellule hôte comprenant un ADN selon l'une quelconque des revendications 1 à 4.

**9.** Cellule hôte selon les revendications 7 ou 8, dans laquelle la cellule est choisie dans le groupe comprenant *E. coli, B. subtilis* et *Streptomyces.*

**10.** Cellule hôte selon la revendication 9, dans laquelle le plasmide, selon la revendication 6, ou l'ADN, selon l'une quelconque des revendications 1 à 4, est intégré dans le génome de la cellule hôte.

**11.** Processus pour la production fermentative d'une protéine de fusion, processus dans lequel :

(a) une molécule d'ADN, selon l'une quelconque des revendications 1 à 4, est exprimée dans une cellule hôte, selon l'une quelconque des revendications 7 à 9 ; et
(b) la protéine de fusion exprimée est isolée.

**12.** Processus selon la revendication 11, dans lequel le surnageant est séparé de la cellule hôte pour isoler la protéine exprimée et la protéine exprimée est isolée à partir du surnageant.

**13.** Processus selon l'une quelconque des revendications 11 ou 12, dans lequel une étape du processus, destinée à concentrer la protéine exprimée dans le surnageant après une précipitation, est choisie dans un groupe comprenant la micro-filtration, une chromatographie à interactions hydrophobes et une chromatographie échangeuse d'ions.

**14.** Processus selon l'une quelconque des revendications 11 à 13, dans lequel l'isolement de la protéine exprimée comprend une étape dans laquelle des composants du milieu de culture ou le surnageant sont précipités alors que la protéine exprimée reste en solution.

**15.** Processus selon l'une quelconque des revendications 11 à 14, dans lequel, après une fermentation, du mercaptan ou du chlorhydrate de cystéine est ajouté au surnageant de la fermentation à un pH de 6 à 9, conduisant à une concentration en groupes SH libres variant de 0,05 à 2,5 mM.

**16.** Processus selon l'une quelconque des revendications 11 à 15, dans lequel, après avoir séparé le surnageant de la fermentation des cellules hôtes, les cellules hôtes sont cultivées de manière répétée dans du milieu frais et la protéine de fusion libérée est isolée à partir de chaque surnageant obtenu lors de la culture.

**17.** Processus selon l'une quelconque des revendications 11 à 15, dans lequel du mercaptan ou du chlorhydrate de cystéine est ajouté au surnageant de la culture de cellules, à un pH de 6 à 9, de sorte qu'une concentration en groupes SH libres variant de 0,05 à 2,5 mM soit atteinte.

**18.** Processus pour la production d'insuline, processus dans lequel :

(a) à partir de la protéine de fusion exprimée, qui est obtenue dans un processus selon l'une quelconque des revendications 11 à 17,
(b) de l'insuline est libérée, par une coupure enzymatique ou chimique, et
(c) elle est isolée.